# EUROPEAN PATENT SPECIFICATION

(11) **EP 2 210 506 B1**
(45) Date of publication and mention of the grant of the patent: **01.05.2013**
(21) Application number: 09166251.0
(22) Date of filing: 23.07.2009
(51) Int. Cl.: A23L 1/30, A23G 3/48, A23G 1/54, A23G 3/36, A61K 36/9064

(54) **Composition against halitosis and for refreshing breath and method for making the same**
Zusammensetzung gegen Halitose und zur Erfrischung des Atems und Verfahren zu deren Herstellung
Composition contre l'halitose et pour rafraichir l'haleine et méthode de production

(30) Priority: 28.07.2008 PT 2008104147
(43) Date of publication of application: 28.07.2010
(73) Proprietor: Corrêa da Silva, Rita Maria, 3700-204 Sao Joao da Madeira (PT)
(72) Inventor: Corrêa da Silva, Rita Maria, 3700-204 Sao Joao da Madeira (PT)
(74) Representative: HOFFMANN EITLE

(56) References cited:
- EP-A2- 0 178 563
- GB-A- 963 780
- JP-A- 2005 154 339
- US-A1- 2008 038 367
- Ivor Hughes: "Cardamomi Semen. U. S. (Br.)Monograph of the USD 21st 1926" Herbdata New Zeeland 2002, XP002554074 Retrieved from the Internet: URL:http://www.herbdatanz.com/cardamom_pic ture_monograph.htm> [retrieved on 2009-11-05]

## Description

### SCOPE OF THE INVENTION

The present invention refers to confectionery products with phytotherapeutic features. In particular it refers to compositions containing *Elettaria cardamomum* (cardamom) seeds, which are useful against halitosis (simultaneously perfuming the breath) and against indigestion, and it also refers to the process used for their preparation.

### BACKGROUND OF THE INVENTION

Since Antiquity plants and their extracts have been used in the treatment of a variety of problems and ailments. Despite significant developments in Western medicine, phytotherapy has been gaining importance in the West, e.g. in view of its benefits in relation to some pathologies (see, for example, the phytotherapy review by Cunha, A.P., Silva, A.P. and Roque, O.R. "plants e produtos vegetais em fitoterapia", Fundação Calouste Gulbenkian, Lisbon, 2003).

Traditional knowledge of some natural plants and herbs, allied to modern technologies, has resulted in new innovative products with phytotherapeutic properties that make the consumption of something natural an experience that is both enjoyable and tasteful, in addition to being potentially medicinal.

Cardamom has 3 varieties; among these, *Elettaria cardamomum* is the species with green-coloured fruits and the greatest potency in terms both of its phytotherapeutic benefits, its flavour and aroma, and this is the species used in the formulations according to this invention.

Since Antiquity different nations have used cardamom for a wide variety of purposes. Egyptians, Romans and Greeks knew how to explore its aromatic and medicinal qualities, and several reports have been found on its applications by them. It is in Indian culture, however, that Cardamom makes its presence intensely felt, both as an aromatic and as a medicinal plant.

The plant *Elettaria cardamomum* (cardamom) can reach a height of 3 metres, and has long tapering leaves and white flowers. Small closed green-coloured pods grow on its erect stalks. Inside each fruit, dark-brown sticky seeds can be found which are very rich in essential oils. However, and with some frequency, these seeds may be greenish yellow and slightly dry or, in some cases, totally dry. The dark seeds, mainly, are hot and slightly sweet to the taste and have an intense camphor-like aroma enjoyed by many. These green pods are picked by hand before ripening and dried immediately. Pods keep the seeds naturally fresh, as these, after being exposed to air, very quickly lose their aromatic properties and, consequently, their phytotherapeutic efficacy.

Besides its therapeutic uses, cardamom nowadays has multiple applications, ranging from patisserie to bakery to its association with teas, coffees and wines, but its most evident application is as a gastronomic spice. As a natural and medicinal product, it is known to have the following properties:
- Excellent nutritional value; rich in proteins and vitamins A, B and C; it contains a minimal percentage of fat and about 10% volatile oils;
- Its seeds are the basis for preparations to combat indigestion and flatulence; because of its carminative properties, it helps to decrease the body's gas and water content;
- It helps increase secretion and excretion of urine and is frequently used as a diuretic, as well in the treatment of conditions associated with the genital-urinary tract, such as nephritis and cystitis;
- It is beneficial in the treatment of such oral conditions as, for example, pharyngitis, sore throats and hoarseness;
- It is also used for its antiseptic, expectorant and laxative powers.
- Because of its oil content, the seed can also be used in cosmetics and perfumery as well as in pharmaceuticals, what makes it a perfect combination to use against halitosis (bad breath).

Today it is known that 95% of the causes of halitosis lie in the oral cavity. Halitosis is usually caused by the lack of or poor tooth and tongue brushing, as well as by the inadequate use of dental floss, by inflammations of the gums and by tooth cavities. Besides these, there are pathological causes, such as rhinitis, sinusitis, pharyngitis, laryngitis and liver or bronco-pulmonary diseases and, also, tobaccoism.

Halitosis constitutes a crucial stigma in most societies, and in some groups the everyday use of products like garlic, onions, etc. is totally banished (despite an awareness of their extraordinary health benefits), for halitosis, to a greater or lesser extent, will have a negative effect on the interpersonal relations on which people depend for their economic and social life. Fundamental benefits are gained through a formula that is capable of, in real time - i.e. relatively quickly -, eliminating the problems created by halitosis, by functioning as a therapeutic and a cohesive factor in these groups.

A number of steps can be taken against the unpleasant odours that develop and tend to proliferate in the oral cavity, including good daily dental care, use of mouthwash and consumption of pastilles or other similar products. Little is usually heard about the use of purely natural products for this purpose, but sodium or potassium salts of chlorophyll obtained through careful alkaline hydrolysis, known as chlorophyllins, are used in some cases. In the long run, however, as these products lead to a discoloration of the mouth mucosa and the teeth, they can only be used in very low concentrations.

Nowadays there is a great demand for products that freshen up the breath and combat halitosis. Orally administered products, preferably natural ones, that provide a quick and effective elimination/reduction of halitosis and, at the same time, perfume the breath are therefore very convenient.

It is also known that bad breath, which is so undesirable, may in many cases originate in the stomach, being caused by difficulties in digesting food, and that some appropriate formulations containing *Elettaria cardamomum* may constitute powerful aids to digestion.

Compositions of this type are known. For example, Japanese patent JP10263064 discloses an oral deodorant containing, among other ingredients, oil extracted from cardamom seeds. The US application US2004/0253192A1 discloses oral products containing cardamom oil to promote oral hygiene and freshen up the breath. These products are presented inter alia as chewing and other gums, confectionery products and mouthwash solutions.

The inventions above, in spite of providing formulations for oral administration aimed at the reduction of halitosis, have the disadvantage of containing cardamom oil in their composition. The process used in the extraction of the oil is a determining factor in its characteristics, not only in view of the possibility of contamination by solvents, but also because fundamental volatile oils are lost in the process. A possible solution to this problem is the use of intact pods or seeds in the preparation.

European patent EP178563 discloses a preparation against bad breath containing cardamom seeds of an unspecified variety. Seeds are coated with a mass of sugar and/or sugar substitutes, to which an aroma such as peppermint may be added. In the two examples presented in this patent, the compositions contain one seed per dragee or 6 seeds per bonbon. The effectiveness of the dragee preparation was tested in several groups of people after the ingestion of substances with a strong smell (onion, garlic, tobacco and beer) and the subsequent administration of 6 dragees. It was found that, on average, even at the end of 2 hours the "bad" breath was significantly noticeable. It only became unnoticeable at the end of 4 hours, after a new intake of 6 dragees, and only in the cases of tobacco and alcohol. This long waiting period is frankly discouraging, as the consumer of these preparations expects much faster and more effective results. It should be stressed that neither in the description nor in the claims of this patent is there any reference to a selection of the type of seeds utilized.

Thus there is an obvious need for new preparations that can solve the technical problem above and, simultaneously, provide unadulterated pleasure when tasted.

The present applicant discovered unexpectedly that the use of selected darker sticky seeds, simultaneous with the rejection of the dry light-coloured ones, often present in the pod - for the preparation of bonbons using chocolate, and their subsequent administration in a specific manner, can lead to really fast and effective results.

The present compositions contain no sugar, but instead, they contain specifically and exclusively sugar substitutes, as well as vegetable fats in diminutive quantities, and all their other carefully selected ingredients are low-fat, for which they are suitable for consumption by people with diabetes, people with raised levels of blood cholesterol and people on low-calorie diets.

In addition, as the base of these compositions is chocolate, which is enjoyed by so many around the world, this will be one more important reason to give great pleasure when tasted.

### SUMMARY OF THE INVENTION

The present invention refers to a composition comprising
(i) dark and sticky seeds of *Elettaria cardamomum* (cardamom) isolated from fruit pods, coated with
(ii) cocoa
for treating and preventing halitosis and indigestion.

The invention also relates to the use of the present composition for the cosmetic treatment and prevention of halitosis.

Furthermore, the invention relates to a process for the preparation of a composition containing *Elettaria cardamomum* (cardamom) comprising the steps of
(i) opening of fruit pods of *Elettaria cardamomum;*
(ii) selecting and collecting dark sticky seeds;
(iii) mixing the selected seeds with the ingredients selected from cocoa and optionally vegetable fat, sweeteners, emulsifiers and flavourings; and
(iv) placing the mixture in appropriate media to obtain the final bonbon.

Preferred embodiments of the present invention are defined in the following description and are further outlined in the appended dependent claims.

### DETAILED DESCRIPTION OR THE INVENTION

The present invention refers to compositions containing *Elettaria cardamomum* (cardamom), which are useful against halitosis and indigestion and for freshening up and perfuming the breath. It also refers to the process used in their preparation.

The present applicant discovered unexpectedly that the use of selected darker sticky seeds, simultaneously with the rejection of the dry light-coloured ones often present in the pod, for the preparation of bonbons using chocolate, and their subsequent administration in a specific manner, can lead to really fast and effective results.

Additionally, the present compositions contain no sugar, but specifically and exclusively sugar substitutes, as well as vegetable fats in diminutive quantities, with all of their other carefully selected ingredients being low-fat. Thus, they are suitable for consumption by people with diabetes, people with raised levels of blood cholesterol and people on low-calorie diets. These compositions are a phytotherapeutical formula to combat halitosis and indigestion. Also to be highlighted is their eminently pleasurable taste, followed by a freshening-up and perfuming of the breath after consumption.

These formulations were conceived with the general health of their consumers in mind, in particular, people with diabetes, with raised levels of blood cholesterol and on low-fat diets. Their minimal calorie input, due to the use of sugar substitutes (sweeteners) and low vegetable fat content, makes their enjoyment available to people who have difficulties metabolising sugars (diabetics) and fats.

In conclusion, thus, these compositions have two objectives:
- Firstly, the phytotherapeutic objective, in the attainment of which the indications provided should be followed;
- Secondly, the exclusive pleasure of savouring a chocolate bonbon with an exquisite cardamom aroma.

It should be noted that, besides 1) the low fat content and the use of only sugar substitutes, the formulations of the present invention introduce, on the one hand, 2) the chocolate component, which most people enjoy, and, on the other hand, 3) the darker sticky seeds, extracted from their hardened wrinkly pods, which are difficult and inconvenient to chew and overload the digestion (hence their exclusion from the product). The careful selection of seeds is due to the fact that the selected seeds are the only ones in which the volatile oil percentage has not been compromised and which, therefore, have the greatest therapeutic potential. It ought to be noted that the contents of one or more pods are added to reinforce the percentage of volatile oils (darker seeds) in the original bonbon, in case the quantity of seeds initially weighed proves insufficient.

The present applicant found that, once opened, pods reveal a random variety of the type of seeds they contain, i.e., there is always the possibility of finding dry seeds (not viable for the product) in them, and a greater possibility of finding half-dry seeds (greenish yellow), which are not so aromatic and, for this reason, not so effective as the seeds selected for the present formulations, which are dark brown and have a potent aroma.

The compositions of the present invention contain dark, sticky seeds of *Elettaria cardamomum* (cardamom) coated in: low-fat powdered cocoa, low-fat cocoa paste, vegetable fat, sweeteners, emulsifiers, preservatives, polish and shine gums (goma de polimento e brilho) and, optionally, flavourings.

More specifically, the compositions include the following percentages relative to total mass:

**Table 1: Ingredients and their percentages relative to total mass**

| Ingredients | Relative percentages (%) |
|---|---|
| Cardamom (dark seeds) | 0.1 - 5 |
| Low-fat powdered cocoa | 40 - 60 |
| Vegetable fat | 5 - 15 |
| Sweeteners | 30 - 50 |
| Emulsifiers | 1 - 3 |
| Preservatives | 0.1 - 1 |
| Polish and shine gum | 0.3 - 1.5 |
| Flavourings | Vestiges |

The compositions are obtained through a process that includes the following steps: (a) opening the fruit pods; (b) selecting and extracting the dark sticky seeds; (c) mixing the selected seeds with selected ingredients from the group comprising cocoa, vegetable fat, sweeteners, emulsifiers, flavourings, and (d) placing the mixture in the appropriate media to obtain the final bonbon.

The final compositions are presented in the form of chocolate bonbons containing whole dark cardamom seeds inside.

The compositions are used for the elimination of halitosis as well as the better functioning of the stomach, they act as a powerful aid to the digestion of foods that are hard to digest, e.g., raw or cooked foods containing large amounts of garlic and onion; alcohol; Eastern foods; fats; tobacco, etc., or to the effective digestion of any types of food (e.g., as fish; fat and hot meats and sauces; fried foods; traditional Portuguese dishes such as papas de serrabulho and feijoada; etc.) that may affect or accentuate pathologies of a gastric nature.

These compositions should be administered after meals, with several intakes, according to the degree of halitosis. Intakes should be repeated as many times as necessary until the complete elimination of any unpleasant odours. Some minutes are necessary between intakes, i.e., about 10 to 15 minutes. As an option, however, and quite apart from their phytotherapeutic roles, formulations can be taken exclusively for the enjoyment of their taste.

### EXAMPLES

### Example 1 - Chocolate bonbon composition 1

Nine *Elettaria cardamomum* cardamom pods are used (from which the darker seeds are extracted). The dark seeds are coated with: low-fat powdered cocoa, low-fat cocoa paste, vegetable fat, sweetener E951 (aspartame), acesulfame K, emulsifier (soy lecithin), preservative (citric acid), gums 2 to 1, and vanilla essence (optional) in the following percentages relatively to total weight:

**Table 2: Ingredients and their percentages relative to total mass of bonbon composition 1**

| Ingredients | Relative percentages (%) |
|---|---|
| Cardamom (only dark seeds) | 1 |
| Low-fat powdered cocoa | 49 |
| Palm oil | 8 |
| Aspartame and acesulfame K | 39 |
| Soy lecithin | 2 |
| Citric acid | 0.3 |
| Polish and shine gum (2 to 1) | 0.7 |
| Vanilla essence | Vestiges |

Product presentation: Chocolate bonbons containing whole cardamom seeds; weight per unity = 10/12 grams.

### Example 2 - Chocolate bonbon composition 2

Seven *Elettaria cardamomum* cardamom pods are used (of which the darker seeds are extracted). The dark seeds are coated with: low-fat powdered cocoa, low-fat cocoa paste, vegetable fat, sweetener E951 (aspartame), acesulfame K, emulsifier (soy lecithin), preservative (citric acid), gums 2 to 1 and vanilla essence (optional) in the following percentages relatively to total weight:

**Table 3: Ingredients and their percentages relative to total mass of bonbon composition 2**

| Ingredients | Relative percentages (%) |
|---|---|
| Cardamom (only dark seeds) | 0.7 |
| Low-fat powdered cocoa | 45.3 |
| Palm oil | 6 |
| Aspartame and acesulfame K | 45 |
| Soy lecithin | 2 |
| Citric acid | 0.3 |
| Polish and shine gum (2 to 1) | 0.7 |
| Vanilla essence | Vestiges |

By way of comparison between the compositions in examples 1 and 2, it should be stressed that the general percentage of sweeteners in composition 2 exceeds the general percentage of sweeteners in composition 1. In spite of this, both have the same phytotherapeutic power against halitosis and indigestion, as well as the same taste potential.

Product presentation: chocolate bonbons containing whole cardamom seeds and powdered cardamom seeds; weight per unit = 10/12 grams.

### Example 3 - Efficacy tests of the formulations

The compositions of the invention were tested to assess their effectiveness in combating the halitosis caused by the consumption of test products such as garlic, onion, tobacco and wine.

Groups of 10 people were submitted to the tests, including 2 people with diabetes and 2 people with high levels of blood cholesterol, the latter 4 people having always been duly monitored. Only one test product was administered to each person. They were provided with 8g garlic, 10g onion, 4 cigarettes or 0.35L wine, or, in the control case, no food. After the ingestion of the food and the consumption of the cigarettes, the halitosis was assessed periodically (at 5-minute intervals) by two independent people. The average waiting time until halitosis was completely eliminated was recorded after each intake of the formulation.

The compositions were administered immediately after the consumption of the test products. Said compositions have been formulated with the objective of combating halitosis and indigestion not only effectively but also more quickly. With this objective, tests were conducted to study the efficacy of the appropriate number of intakes until the total elimination of halitosis in the shortest possible time had been verified. The same group, thus, was tested further: Composition 1 - up to 3 intakes were administered, the first after the ingestion of the test food; the second about 10 minutes later; and a third 15 minutes thereafter; composition 2 - administration of 4 + 2 intakes (the latter 2 optional), the first after the ingestion of the test food; the second and third at intervals of approximately 10 minutes; and a fourth after approximately 15 minutes. The optional intakes can be administered according to the wishes of those consuming the formulations.

**Table 3: Efficacy tests of composition 1 and 2 with the respective waiting time (minutes) until total elimination of halitosis**

| **Formulation 1** | | | **Formulation 2** | |
|---|---|---|---|---|
| Test with only 1 intake | | | Test with only 1 intake | |
| Food tested | Waiting | | Food tested | Waiting |
| Garlic | - | | Garlic | - |
| Onion | - | | Onion | - |
| Tobacco | 60 | | Tobacco | 60 |
| Wine | 60 | | Wine | 60 |
| | | | | |
| Test with 2 intakes | | | Test with 2 intakes | |
| Garlic | 80 | | Garlic | - |
| Onion | 60 | | Onion | - |
| Tobacco | 35 | | Tobacco | 40 |
| Wine | 30 | | Wine | 35 |
| | | | | |
| Test with 3 intakes | | | Test with 3 intakes | |
| Garlic | 35 | | Garlic | 60 |
| Onion | 25 | | Onion | 45 |
| Tobacco | 15 | | Tobacco | 30 |
| Wine | 10 | | Wine | 25 |
| | | | | |
| | | | Test with 4 intakes | |
| | | | Garlic | 30 |
| | | | Onion | 20 |
| | | | Tobacco | 10 |
| | | | Wine | 5 |

There were no problems of intolerance to the product on the part of any individual. All enjoyed it enthusiastically and reported a clear improvement in their digestion after the taking it. As examples of the elimination of halitosis, it was found that, with 3 intakes spaced at 10- and 15-minute intervals, bad breath was totally eliminated after 10 minutes in the case of wine, and after 35 minutes in the case of garlic.

It is concluded, therefore, that regarding composition 1, with 3 chocolate bonbons taken at intervals of 10 and 15 minutes, it is possible to eliminate a strong garlic odour completely at the end of 35 minutes. Regarding composition 2, with 4 bonbons taken at intervals of 10, 10 and 15 minutes, it is possible to eliminate a strong garlic odour at the end of 30 minutes.

### Example 4 - Dosage scheme

To be totally effective, the compositions of the present invention should be administered according to the following scheme: suck and chew the whole bonbons well, after main meals, specially the seeds (a most important ingredient), and then swallow them slowly and confidently.

**Table 4: Chronology of administration of composition 1 (Example 1)**

| Intake n° | Time for administration (minutes) | Total time for odour elimination (minutes) |
|---|---|---|
| 1 | 0 | - |
| 2 | 10 | 80-30 |
| 3 | 25 | 35-10 |

**Table 5: Chronology of administration of composition 2 (Example 2)**

| Intake n° | Time for administration (minutes) | Total time for odour elimination (minutes) |
|---|---|---|
| 1 | 0 | - |
| 2 | 10 | - |
| 3 | 20 | 60-25 |
| 4 | 35 | 30-5 |
| 5 + 6 | * | * |

| | | |
|---|---|---|
| * Intakes for pleasure (optional) | | |

## Claims

1. Composition comprising
(i) dark and sticky seeds of *Elettaria cardamomum* (cardamom) isolated from fruit pods,
coated with
(ii) cocoa
for treating and preventing halitosis and indigestion.

2. Composition according to claim 1, wherein cocoa is a low-fat powdered cocoa or a low-fat cocoa paste.

3. Composition according to claim 1 or 2, wherein the dark and sticky seeds of *Elettaria cardamomum* (cardamom) isolated from fruit pods are further coated with ingredients selected from vegetable fat, sweeteners, emulsifiers, preservatives, or flavourings.

4. Composition according to claim 1 comprising,
(i) 0.1- 5 %, based on the total weight of the composition, of dark and sticky seeds of *Elettaria cardamomum* (cardamom) isolated from fruit pods,
coated with
(ii)
| | |
|---|---|
| 40-60% | low-fat powdered cocoa; |
| 5-15% | vegetable fat; |
| 30-50% | sweeteners selected from aspartame and acesulfame K; |
| 1-3% | emulsifiers; |
| 0.1-1% | preservatives; |
| 0.3-1.5% | polish and shine gum (*goma de polimento e brilho*); and |
vestigial quantities of flavourings;
each based on the total weight of the composition.

5. The composition of claim 4, comprising
| | |
|---|---|
| 0.7% | dark seeds of *Elettaria cardamomum;* |
| 45.3% | low-fat powdered cocoa; |
| 6% | vegetable fat; |
| 45% | sweeteners selected from aspartame and acesulfame K; |
| 2% | emulsifiers; |
| 0.3% | preservatives; |
| 0.7% | polish and shine gum (*goma de polimento e brilho*); and |
vestigial quantities of vanilla essence, each based on the total weight of the composition.

6. The composition of claim 4, comprising
| | |
|---|---|
| 1% | dark seeds of *Elettaria cardamomum;* |
| 49% | low-fat powdered cocoa; |
| 8% | palm oils; |
| 39% | sweeteners selected from aspartame and acesulfame K; |
| 2% | soy lecithin; |
| 0.3% | citric acid; |
| 0.7% | polish and shine gum (*goma de polimento e brilho*); and |
vestigial quantities of vanilla essence each based on the total weight of the composition.

7. Use of a composition comprising
(i) dark and sticky seeds of *Elettaria cardamomum* (cardamom) isolated from fruit pods,
coated with
(ii) cocoa
for cosmetic treatment and prevention of halitosis.

8. The use of claim 7, wherein cocoa is a low-fat powdered cocoa or a low-fat cocoa paste.

9. The use of claim 7 or 8, wherein the composition further comprises vegetable fat, sweeteners, emulsifiers, preservatives, polish and shine gum (*goma de polimento* e *brilho),* and flavourings.

10. The use of claim 7, wherein the composition comprises
(i) 0.1- 5 %, based on the total weight of the composition, of dark and sticky seeds of *Elettaria cardamomum* (cardamom) isolated from fruit pods,
coated with
(ii)
| | |
|---|---|
| 40-60% | low-fat powdered cocoa; |
| 5-15% | vegetable fat; |
| 30-50% | sweeteners selected from aspartame and acesulfame K; |
| 1-3% | emulsifiers; |
| 0.1-1% | preservatives; |
| 0.3-1.5% | polish and shine gum (*goma de polimento e brilho*); and |
vestigial quantities of flavourings;
each based on the total weight of the composition.

11. The use of claim 10, wherein the composition comprises
| | |
|---|---|
| 0.7% | dark seeds of *Elettaria cardamomum;* |
| 45.3% | low-fat powdered cocoa; |
| 6% | vegetable fat; |
| 45% | sweeteners selected from aspartame and acesulfame K; |
| 2% | emulsifiers; |
| 0.3% | preservatives; |
| 0.7% | polish and shine gum (*goma de polimento e brilho*); and |
vestigial quantities of vanilla essence,
each based on the total weight of the composition.

12. The use of claim 10, wherein the composition comprises
| | |
|---|---|
| 1% | dark seeds of *Elettaria cardamomum;* |
| 49% | low-fat powdered cocoa; |
| 8% | palm oils; |
| 39% | sweeteners selected from aspartame and acesulfame K; |
| 2% | soy lecithin; |
| 0.3% | citric acid; |
| 0.7% | polish and shine gum (*goma de polimento e brilho*); and |
vestigial quantities of vanilla essence
each based on the total weight of the composition.

13. Process for the preparation of a composition containing *Elettaria cardamomum* (cardamom) comprising the steps of
(i) opening of fruit pods of *Elettaria cardamomum;*
(ii) selecting and collecting dark sticky seeds;
(iii) mixing the selected seeds with the ingredients selected from cocoa and optionally vegetable fat, sweeteners, emulsifiers and flavourings; and
(iv) placing the mixture in appropriate media to obtain the final bonbon.

## Patentansprüche

1. Zusammensetzung, umfassend:
(i) dunkle und klebrige Samen von Elettaria cardamomum (Kardamom), isoliert aus Fruchtschoten,
beschichtet mit
(ii) Kakao
zur Behandlung und Prävention von Mundgeruch und Verdauungsstörungen.

2. Zusammensetzung nach Anspruch 1, wobei der Kakao fettarmes Kakaopulver oder fettarme Kakaomasse darstellt.

3. Zusammensetzung nach einem der Ansprüche 1 oder 2, wobei die dunklen und klebrigen Samen von Elettaria cardamomum (Kardamom), isoliert aus Fruchtschoten, ferner mit Inhaltsstoffen ausgewählt aus Pflanzenfett, Süßungsmitteln, Emulgatoren, Konservierungsmitteln oder Aromastoffen beschichtet werden.

4. Zusammensetzung nach Anspruch 1, umfassend:
(i) 0,1-5 %, bezogen auf das Gesamtgewicht der Zusammensetzung, dunkle und klebrige Samen von Elettaria cardamomum (Kardamom), isoliert aus Fruchtschoten,
beschichtet mit
(ii)
| | |
|---|---|
| 40-60% | fettarmem Kakaopulver; |
| 5-15% | Pflanzenfett; |
| 30-50% | Süßungsmittel ausgewählt aus Aspartam und Acesulfam K; |
| 1-3% | Emulgatoren; |
| 0,1-1% | Konservierungsmittel; |
| 0,3-1,5% | Polier- und Glanzgummi (goma de polimento e brilho); und |
geringen Mengen an Aromastoffen;
wobei jeder Bestandteil auf das Gesamtgewicht der Zusammensetzung bezogen ist.

5. Zusammensetzung nach Anspruch 4, umfassend:
| | |
|---|---|
| 0,7% | dunkle Samen von Elettaria cardamomum; |
| 45,3% | fettarmes Kakaopulver; |
| 6% | Pflanzenfett; |
| 45% | Süßungsmittel ausgewählt aus Aspartam und Acesulfam K; |
| 2% | Emulgatoren; |
| 0,3% | Konservierungsmittel; |
| 0,7% | Polier- und Glanzgummi (goma de polimento e brilho); und |
geringe Mengen an Vanilleessenz,
wobei jeder Bestandteil auf das Gesamtgewicht der Zusammensetzung bezogen ist.

6. Zusammensetzung nach Anspruch 4, umfassend:
| | |
|---|---|
| 1% | dunkle Samen von Elettaria cardamomum; |
| 49% | fettarmes Kakaopulver; |
| 8% | Palmöle; |
| 39% | Süßungsmittel ausgewählt aus Aspartam und Acesulfam K; |
| 2% | Sojalecithin; |
| 0,3% | Zitronensäure; |
| 0,7% | Polier- und Glanzgummi (goma de polimento e brilho); und |
geringe Mengen an Vanilleessenz,
wobei jeder Bestandteil auf das Gesamtgewicht der Zusammensetzung bezogen ist.

7. Verwendung einer Zusammensetzung, umfassend:
(i) dunkle und klebrige Samen von Elettaria cardamomum (Kardamom), isoliert aus Fruchtschoten,
beschichtet mit
(ii) Kakao
zur kosmetischen Behandlung und Prävention von Mundgeruch.

8. Verwendung nach Anspruch 7, wobei der Kakao fettarmes Kakaopulver oder fettarme Kakaomasse darstellt.

9. Verwendung nach Anspruch 7 oder 8, wobei die Zusammensetzung ferner Pflanzenfett, Süßungsmittel, Emulgatoren, Konservierungsmittel, Polier- und Glanzgummi (goma de polimento e brilho) und Aromastoffe umfasst.

10. Verwendung nach Anspruch 7, wobei die Zusammensetzung umfasst:
(i) 0,1-5 %, bezogen auf das Gesamtgewicht der Zusammensetzung, dunkle und klebrige Samen von Elettaria cardamomum (Kardamom), isoliert aus Fruchtschoten,
beschichtet mit
(ii)
| | |
|---|---|
| 40-60% | fettarmem Kakaopulver; |
| 5-15% | Pflanzenfett; |
| 30-50% | Süßungsmittel ausgewählt aus Aspartam und Acesulfam K; |
| 1-3% | Emulgatoren; |
| 0,1-1% | Konservierungsmitteln; |
| 0,3-1,5% | Polier- und Glanzgummi (goma de polimento e brilho); und |
geringen Mengen an Aromastoffen;
wobei jeder Bestandteil auf das Gesamtgewicht der Zusammensetzung bezogen ist.

11. Verwendung nach Anspruch 10, wobei die Zusammensetzung umfasst:
| | |
|---|---|
| 0,7% | dunkle Samen von Elettaria cardamomum; |
| 45,3% | fettarmes Kakaopulver; |
| 6% | Pflanzenfett; |
| 45% | Süßungsmittel ausgewählt aus Aspartam und Acesulfam K; |
| 2% | Emulgatoren; |
| 0,3% | Konservierungsmittel; |
| 0,7% | Polier- und Glanzgummi (goma de polimento e brilho); und |
geringe Mengen an Vanilleessenz,
wobei jeder Bestandteil auf das Gesamtgewicht der Zusammensetzung bezogen ist.

12. Verwendung nach Anspruch 10, wobei die Zusammensetzung umfasst:
| | |
|---|---|
| 1% | dunkle Samen von Elettaria cardamomum; |
| 49% | fettarmes Kakaopulver; |
| 8% | Palmöle; |
| 39% | Süßungsmittel ausgewählt aus Aspartam und Acesulfam K; |
| 2% | Sojalecithin; |
| 0,3% | Zitronensäure; |
| 0,7% | Polier- und Glanzgummi (goma de polimento e brilho); und |
geringe Mengen an Vanilleessenz,
wobei jeder Bestandteil auf das Gesamtgewicht der Zusammensetzung bezogen ist.

13. Verfahren zur Herstellung einer Zusammensetzung, die Elettaria cardamomum (Kardamom) enthält, wobei das Verfahren die folgenden Schritte umfasst:
(i) Öffnen der Fruchtschoten von Elettaria cardamomum;
(ii) Auswählen und Sammeln der dunklen, klebrigen Samen;
(iii) Mischen der ausgewählten Samen mit den Bestandteilen ausgewählt aus Kakao und gegebenenfalls Pflanzenfett, Süßungsmitteln, Emulgatoren und Aromastoffen; und
(iv) Einbringung der Mischung in geeignete Medien, um das endgültige Bonbon zu gewinnen.

## Revendications

1. Composition comprenant
(i) des graines sombres et collantes de l'*Elettaria cardamomum* (cardamome) isolées à partir de gousses de fruit,
enrobées avec
(ii) du cacao
destinée à traiter et à prévenir l'halitose et l'indigestion.

2. Composition selon la revendication 1, dans laquelle le cacao est du cacao en poudre fortement dégraissé ou une pâte de cacao fortement dégraissé.

3. Composition selon la revendication 1 ou 2, dans laquelle les graines sombres et collantes de l'*Elettaria cardamomum* (cardamome) isolées à partir de gousses de fruit sont en outre enrobées d'ingrédients choisis parmi la graisse végétale, des édulcorants, des émulsifiants, des agents de conservation, ou des arômes.

4. Composition selon la revendication 1, comprenant,
(i) 0,1-5%, par rapport au poids total de la composition, de graines sombres et collantes de l'*Elettaria cardamomum* (cardamome) isolées à partir de gousses de fruit,
enrobées avec
(ii) 40-60% de cacao en poudre fortement dégraissé ;
5-15% de graisse végétale ;
30-50% d'édulcorants choisis parmi l'aspartame et l'acésulfame K ;
1-3% d'émulsifiants ;
0,1-1% d'agents de conservation ;
0,3-1,5% de gomme de polissage et de brillance (goma de polimento e brilho) ; et
des quantités résiduelles d'arômes ;
chaque pourcentage étant basé sur le poids total de la composition.

5. Composition de la revendication 4, comprenant
0,7% de graines sombres de l'*Elettaria cardamomum* ;
45,3% de cacao en poudre fortement dégraissé ;
6% de graisse végétale ;
45% d'édulcorants choisis parmi l'aspartame et l'acésulfame K ;
2% d'émulsifiants ;
0,3% d'agents de conservation ;
0,7% de gomme de polissage et de brillance (goma de polimento e brilho) ; et
des quantités résiduelles d'essence de vanille, chaque pourcentage étant basé sur le poids total de la composition.

6. Composition de la revendication 4, comprenant
1% de graines sombres de l'*Elettaria cardamomum* ;
49% de cacao en poudre fortement dégraissé ;
8% d'huiles de palme ;
39% d'édulcorants choisis parmi l'aspartame et l'acésulfame K ;
2% de lécithine de soja ;
0,3% d'acide citrique ;
0,7% de gomme de polissage et de brillance (goma de polimento e brilho) ; et
des quantités résiduelles d'essence de vanille
chaque pourcentage étant basé sur le poids total de la composition.

7. Utilisation d'une composition comprenant
(i) des graines sombres et collantes de l'*Elettaria cardamomum* (cardamome) isolées à partir de gousses de fruit,
enrobées avec
(ii) du cacao
pour un traitement cosmétique et la prévention de l'halitose.

8. Utilisation de la revendication 7, dans laquelle le cacao est du cacao en poudre fortement dégraissé ou une pâte de cacao fortement dégraissé.

9. Utilisation de la revendication 7 ou 8, dans laquelle la composition comprend en outre de la graisse végétale, des édulcorants, des émulsifiants, des agents de conservation, une gomme de polissage et de brillance (goma de polimento e brilho), et des arômes.

10. Utilisation de la revendication 7, dans laquelle la composition comprend
(i) 0,1-5%, par rapport au poids total de la composition, de graines sombres et collantes de l'*Elettaria cardamomum* (cardamome) isolées à partir de gousses de fruit,
enrobées avec
(ii) 40-60% de cacao en poudre fortement dégraissé ;
5-15% de graisse végétale ;
30-50% d'édulcorants choisis parmi l'aspartame et l'acésulfame K ;
1-3% d'émulsifiants ;
0,1-1% d'agents de conservation ;
0,3-1,5% de gomme de polissage et de brillance (goma de polimento e brilho) ; et
des quantités résiduelles d'arômes ;
chaque pourcentage étant basé sur le poids total de la composition.

11. Utilisation de la revendication 10, dans laquelle la composition comprend
0,7% de graines sombres de l'*Elettaria cardamomum* ;
45,3% de cacao en poudre fortement dégraissé ;
6% de graisse végétale ;
45% d'édulcorants choisis parmi l'aspartame et l'acésulfame K ;
2% d'émulsifiants ;
0,3% d'agents de conservation ;
0,7% de gomme de polissage et de brillance (goma de polimento e brilho) ; et
des quantités résiduelles d'essence de vanille,
chaque pourcentage étant basé sur le poids total de la composition.

12. Utilisation de la revendication 10, dans laquelle la composition comprend
1% de graines sombres de l'*Elettaria cardamomum* ;
49% de cacao en poudre fortement dégraissé ;
8% d'huiles de palme ;
39% d'édulcorants choisis parmi l'aspartame et l'acésulfame K ;
2% de lécithine de soja ;
0,3% d'acide citrique ;
0,7% de gomme de polissage et de brillance (goma de polimento e brilho) ; et
des quantités résiduelles d'essence de vanille
chaque pourcentage étant basé sur le poids total de la composition.

13. Processus destiné à la préparation d'une composition contenant l'*Elettaria cardamomum* (cardamome) comprenant les étapes consistant
(i) à ouvrir des gousses de fruit de l'*Elettaria cardamomum* ;
(ii) à sélectionner et à récupérer des graines collantes sombres ;
(iii) à mélanger les graines sélectionnées avec les ingrédients choisis parmi le cacao et facultativement de la graisse végétale, des édulcorants, des émulsifiants et des arômes ; et
(iv) à placer le mélange dans un milieu approprié afin d'obtenir le bonbon final.
